# EUROPÄISCHE PATENTSCHRIFT

(11) **EP 0 641 377 B1**
(45) Veröffentlichungstag und Bekanntmachung des Hinweises auf die Patenterteilung: **11.09.1996**
(21) Anmeldenummer: 93909940.4
(22) Anmeldetag: 11.05.1993
(51) Int. Cl.: C11D 1/83

(54) **MILDE TENSIDGEMISCHE**
MILD SURFACTANT MIXTURE
MELANGES DE TENSIOACTIFS DOUX

(30) Priorität: 18.05.1992 US 884685
(43) Veröffentlichungstag der Anmeldung: 08.03.1995
(73) Patentinhaber: Henkel Kommanditgesellschaft auf Aktien, 40191 Düsseldorf (DE)
(72) Erfinder: HENSEN, Hermann, D-5657 Haan (DE); TESMANN, Holger, D-4000 Düsseldorf 13 (DE); KRÄCHTER, Hans-Udo, D-4630 Bochum (DE); BUSCH, Peter, D-4006 Erkrath-Unterbach (DE); STUHRMANN, Dagmar, Sosua Poerto Plata (DO); SALKA, Barry, A., Fair Lawn, NJ 07410 (US)
(86) Internationale Anmeldenummer: EP9301148
(87) Internationale Veröffentlichungsnummer: WO9323512

(56) Entgegenhaltungen:
- EP-A- 0 409 005
- EP-A- 0 486 786
- WO-A-90/02164
- WO-A-91/04313
- WO-A-91/15192
- WO-A-92/21742
- US-A- 4 483 787
- CHEMICAL ABSTRACTS, vol. 115, no. 24, 16. Dezember 1991, Columbus, Ohio, US; abstract no. 258774b, Seite 152

## Beschreibung

### Gebiet der Erfindung

Die Erfindung betrifft milde Tensidgemische enthaltend Alkyl - und/oder Alkenyloligoglucoside verschiedener Kettenlänge und Alkylethersulfate sowie deren Verwendung zur Herstellung oberflächenaktiver Mittel.

### Stand der Technik

An Mittel und Zubereitungen, die der Reinigung oder Pflege von Körper und Haaren dienen oder die in anderer Weise mit der menschlichen Haut in Kontakt treten, werden hohe Anforderungen an das Schaum- und Reinigungsvermögen sowie an die Hautverträglichkeit gestellt. Aufgrund ihrer guten anwendungstechnischen Eigenschaften wird zur Herstellung derartiger Produkte häufig auf anionische Tenside zurückgegriffen. Eine besondere Bedeutung kommt dabei den Alkylethersulfaten zu, die sich durch ein starkes Schaumvermögen, gute Reinigungsleistung sowie geringe Fett- und Härteempfindlichkeit auszeichnen. Obschon Alkylethersulfate dermatologisch günstig beurteilt werden, ist die Hautverträglichkeit dieser Stoffe jedoch für manche Zwecke noch nicht ausreichend.

Es ist bekannt, daß man die Hautverträglichkeit anionischer Tenside verbessern kann, indem man sie mit amphoteren oder zwitterionischen Tensiden kombiniert. In vielen Fällen wird jedoch für solche Mischungen nur dann eine ausreichende Hautverträglichkeit beobachtet, wenn der Anteil der amphoteren oder zwitterionischen Tenside an den Mischungen mehr als 25 Gew.-% beträgt. Desweiteren führt die Abmischung von anionischen mit amphoteren oder zwitterionischen Tensiden häufig zu einer Abnahme des Schaumvermögens.

Es ist ferner bekannt, daß sich die Hautverträglichkeit von Aniontensiden verbessern läßt, wenn man diese in Form ihrer Magnesiumsalze einsetzt [**Ärztl.Kosmetol., 19, 208 (1989)**]. Wegen ihrer meist geringen Wasserlöslichkeit ist die Verwendung von Aniontensid-Magnesiumsalzen zur Herstellung wäßriger Produkte nur eingeschränkt möglich.

Ein weiterer Weg zur Verbesserung der Hautverträglichkeit von Aniontensiden besteht schließlich darin, sie mit Eiweiß-Abbauprodukten zu kombinieren [**Seifen-Öle-Fette-Wachse, 108, 177 (1982)**]. Derartige Proteinhydrolysate verfügen jedoch über keine oberflächenaktiven Eigenschaften und führen in der Regel ebenfalls zu einer Verschlechterung der anwendungstechnischen Eigenschaften.

Milde Tensidgemische auf Basis von Ethersulfaten sind aus dem Stand der Technik bekannt. Stellvertretend für das umfangreiche Schrifttum sei auf Mischungen von Alkylethersulfaten mit Alkyl- und/oder Alkenylglucosiden und amphoteren bzw. zwitterionischen Tenside [**WO 91/15 192** (Henkel)] oder innenständigen Alkylethersulfonaten [**EP-A-0 455 657** (Henkel)] verwiesen.

Aus der Europäischen Patentschrift **EP-B1-0 070 0 74** (Procter & Gamble) sind schäumende Tensidmischungen bekannt, die C₈-C₁₈-Alkyloligoglucoside und anionische Tenside, darunter auch Alkylethersulfate enthalten können. In den Beispielen 20 und 21 werden beispielsweise wäßrige Mischungen offenbart, die neben einem Alkyloligoglucosid der Kettenlänge C_{12/13} (durchschnittlicher Polymerisationsgrad DP = 1,7) und einem C_{12/13}-Alkyl-0,8EO-ethersulfat-Ammoniumsalz überwiegend andere Tenside, wie beispielsweise Alkylbenzolsulfonate und Olefinsulfonate sowie als weitere Bestandteile Lösungsvermittler (Ethanol, Xylolsulfonat) enthalten. Mischungen einer solchen Zusammensetzung kommen aus dermatologischen Gründen für den angestrebten Einsatz nicht in Betracht. Zudem ist die Verwendung von Lösungsvermittlern ebenfalls unerwünscht.

Die Aufgabe der Erfindung bestand somit darin, neue milde Tensidgemische auf der Basis von Alkylethersulfaten zur Verfügung zu stellen, die frei von den geschilderten Nachteilen sind.

### Beschreibung der Erfindung

Gegenstand der Erfindung sind milde Tensidgemische, enthaltend
a) 10 bis 40 Gew.-% eines Alkyl- und/oder Alkenyloligoglucosids der Formel (I),

   R¹-O-[G]ₚ (I)

   in der
   - R¹: für einen Alkyl- und/oder Alkenylrest mit 8 bis 11 Kohlenstoffatomen,
   - [G]: für einen Glucoserest und
   - p: für Zahlen von 1 bis 10 steht,
b) 10 bis 40 Gew.-% eines Alkyl- und/oder Alkenyloligoglucosids der Formel **(II)**,

   R²-O-[G]ₚ (II)

   in der
   - R²: für einen Alkyl- und/oder Alkenylrest mit 12 bis 22 Kohlenstoffatomen,
   - [G]: für einen Glucoserest und
   - P: für Zahlen von 1 bis 10 steht und
c) 80 bis 20 Gew.-% eines Alkylethersulfats der Formel **(III)**,

   R³-(OCH₂CH₂)ₙO-SO₃M (III)

   in der
   - R³: für einen Alkyl- und/oder Alkenylrest mit 6 bis 22 Kohlenstoffatomen,
   - M: für ein Alkali-, Erdalkali-, Ammonium- und/oder Alkanolammoniumion und
   - n: für Zahlen von 1 bis 20
   steht.

Überraschenderweise wurde gefunden, daß Tensidmischungen, die neben Alkylethersulfaten verschiedene Alkyloligoglucosidtypen, nämlich solche einer Kettenlänge C₈-C₁₁ und solche einer Kettenlänge C₁₂-C₂₂, innerhalb bestimmter Mischungsverhältnisse nicht nur ein Irritationsmimimum zeigen, sondern gleichzeitig ein Maximum der Schaumstabilität aufweisen. Die Tensidgemische sind zudem ohne Zusatz von Lösungsvermittlern klar wasserlöslich. In Gegenwart von Hautfett wird gegenüber herkömmlichen Alkylethersulfat/Alkyloligoglucosid-Mischungen eine synergistische Verbesserung des Schaumvermögens und eine geringere Beeinflussung der Hautfeuchte beobachtet. Weitere Vorteile ergeben sich hinsichtlich der Reißfestigkeit von Haarbündeln und der Trockenkämmbarkeit von Haarsträhnen.

**Alkyl- und Alkenyloligoglucoside**, die die Komponenten a) und b) der erfindungsgemäßen Mischun bilden, stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Indexzahl p in der allgemeinen Formel **(I)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkylrest R¹ kann sich von primären Alkoholen mit 8 bis 11, vorzugsweise 8 bis 10 Kohlenstoffatomen ableiten. Typische Beispiele sind Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R² kann sich entsprechend von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Bei den **Alkylethersulfaten** der Komponente c) handelt es sich um eine wohlbekannte Klasse anionischer Tenside, die üblicherweise durch Umsetzung von Fettalkoholen mit gasförmigem Schwefeltrioxid bzw. Chlorsulfonsäure und nachfolgender Neutralisation mit wäßrigen Basen hergestellt werden. Als Bestandteile der erfindungagemäßen Tensidgemische kommen Alkylethersulfate in Betracht, die sich von gesättigten oder einfach ungesättigten Alkoholethoxylaten mit 6 bis 22 Kohlenstoffatomen in der Alkylkette und 1 bis 20 Ethylenoxideinheiten in der Polyetherkette ableiten. Vorzugsweise werden Alkylethersulfate der Formel **(III)** eingesetzt, in der R³ für Alkylreste mit 12 bis 18 Kohlenstoffatomen, M für Natrium und/oder Magnesium und n für Zahlen von 2 bis 7 steht.

In einer bevorzugten Ausführungsform der Erfindung kommen Alkylethersulfate zum Einsatz, die über eine eingeengte Homologenverteilung verfügen, wie sie beispielsweise in der **WO 91/05 764** (Henkel) beschrieben sind.

Aus anwendungstechnischer und dermatologischer Sicht haben sich Tensidgemische als besonders vorteilhaft erwiesen, die die Komponenten a) und b) im Gewichtsverhältnis 1 : 1 bis 4 : 1, vorzugsweise 1 : 1 bis 2 **:** 1 sowie die Komponenten (a+b) und c) im Gewichtsverhältnis 4 : 1 bis 1 : 4, vorzugsweise 3 : 1 bis 1 : 3 enthalten.

Zur Herstellung der erfindungsgemäßen Tensidgemische ist es ausreichend, die einzelnen Komponenten, gegebenenfalls bei leicht erhöhter Temperatur von 25 bis 40°C unter Rühren zusammenzugeben, wobei die Reihenfolge unkritisch ist; eine chemische Reaktion findet nicht statt. Es ist ferner möglich, die Komponenten in wasserfreier oder hochkonzentrierter Form vorzulegen und anschließend mit Wasser auf eine Anwendungskonzentration von 1 bis 50, vorzugsweise 15 bis 30 Gew.-% zu verdünnen oder aber unmittelbar verdünnte wäßrige Lösungen für die Herstellung der Mischungen zu verwenden.

Die hautschonenden Eigenschaften der erfindungsgemäßen Mittel kommen besonders dann zur Geltung, wenn diese so formuliert werden, daß sie einen pH-Wert in der Nähe des Neutralpunktes aufweisen. Mittel mit pH-Werten im Bereich von 5,5 bis 7,5, insbesondere 5,5 bis 6,5 sind daher bevorzugt.

Für die Herstellung von Endformulierungen können die erfindungsgemäßen Tensidgemische weitere übliche Bestandteile von kosmetischen Zubereitungen wie beispielsweise weitere hautverträgliche Tenside, Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten.

Als weitere milde Tenside kommen beispielsweise innenständige Alkylethersulfonate, Hydroxymischethersulfate, Ölsäuresulfonate, Alkylsulfosuccinate, Isethionate, Tauride, Sarcosinate, Alkylethercarbonsäuren, Alkyl(ether)phosphate, Alkyloligoglucosidsulfate, Alkylamidobetaine, Aminopropionate, Imidazoliniumbetaine und/oder Sulfobetaine in Betracht.

Übliche Ölkomponenten sind Substanzen wie Paraffinöl, Pflanzenöle, Fettsäureester, Squalan und 2-Octyldodecanol, während als Fette und Wachse beispielsweise Walrat, Bienenwachs, Montanwachs, Paraffin und Cetylstearylalkohol Verwendung finden.

Als Überfettungsmittel können Substanzen wie beispielsweise polyethoxylierte Lanolinderivate, Lecithinderivate und Fettsäurealkanolamide verwendet werden, wobei die letzteren gleichzeitig als Schaumstabilisatoren dienen.

Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid.

Unter biogenen Wirkstoffen sind beispielsweise Pflanzenextrakte, Eiweißhydrolysate und Vitaminkomplexe zu verstehen.

Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich beispielsweise Formaldehydlösung, p-Hydroxybenzoat oder Sorbinsäure.

Als Perlglanzmittel kommen beispielsweise Glycoldistearinsäureester wie Ethylenglycoldistearat, aber auch Fettsäuremonoglycolester in Betracht.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984**, zusammengestellt sind. Diese Farbstoffe werden üblicherweise in Konzentrationen von 0,001 bis 0,1 Gew.-%, bezogen auf die gesamte Mischung, eingesetzt.

### Gewerbliche Anwendbarkeit

Die erfindungsgemäßen Tensidgemische zeichnen sich gegenüber herkömmlichen Mischungen von Alkyloligoglucosiden und Alkylethersulfaten durch eine verbesserte dermatologische Verträglichkeit und eine höhere Schaumstabilität aus. In Gegenwart von Hautfett wird eine weitere synergistische Verstärkung des Schaumverhaltens beobachtet. Die erfindungsgemäßen Mischungen beeinflussen die Hautfeuchte in geringerem Maße und zeigen weitere Vorteile im Hinblick auf die Reißfestigkeit von Haarbündeln sowie die Trockenkämmbarkeit von Haarsträhnen.

Ein weiterer Gegenstand der Erfindung betrifft daher die Verwendung der Tensidgemische zur Herstellung von oberflächenaktiven Mitteln, die mit der menschlichen Haut in Kontakt treten, insbesondere manuellen Geschirrspülmitteln sowie kosmetischen Produkten, wie beispielsweise Haarshampoos, Schaumbädern oder Körperreinigungsmitteln, in denen sie in Mengen von 1 bis 60, vorzugsweise 5 bis 50 und insbesondere 10 bis 30 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Eingesetzte Tenside

A1) Plantaren^{(R)} APG 200:
   C_{8/10}-Alkyloligoglucosid (DP = 1,3),
   Aktivsubstanzgehalt : 30 Gew.-%
   Verkaufsprodukt Fa.Henkel KGaA, Düsseldorf, FRG.
A2) Plantaren^{(R)} APG 600:
   C_{12/14}-Alkyloligoglucosid (DP = 1,3),
   Aktivsubstanzgehalt : 30 Gew.-%
   Verkaufsprodukt Fa.Henkel KGaA, Düsseldorf, FRG.
B1) Texapon^{(R)} N25:
   C_{12/14}-Kokosfettalkohol-2EO-ethersulfat-Na-Salz,
   Aktivsubstanzgehalt : 25 Gew.-%
   Verkaufsprodukt Fa.Henkel KGaA, Düsseldorf, FRG.

### II. Dermatologische Untersuchungen

Die dermatologische Beurteilung der Tensidmischungen erfolgte mit Hilfe des Duhring-Kammer-Tests (DKT). Hierbei handelt es sich um eine okklusiv stattfindende, mehrfache Applikation verdünnter wäßriger Lösungen der Testgemische auf die Beugeseite der Unterarme von Probanden. Am ersten Tag betrug die Expositionszeit 24 h, an den folgenden Tagen jeweils 6 h. Die verdünnten Testsubstanzen wurden in einer Menge von 0,1 ml mit Hilfe der Duhring-Kammer appliziert und die Hautreaktion nach der 5. Exposition beurteilt. Zu Einzelheiten vgl. **Fette, Seifen, Anstrichmitt., 83, 33 (1981)**.

**Tab. 1:**

| Hautverträglichkeit (Erythrem) | | | | |
|---|---|---|---|---|
| Bsp. | A1 : | A2 : | B1 | DKT-Reizscore %- rel |
| 1 | 12 : | 12 : | 76 | 68 |
| 2 | 25 : | 25 : | 50 | 46 |
| 3 | 24 : | 38 : | 38 | 20 |
| V1 | 0 : | 0 : | 100 | 100 |

## Patentansprüche

1. Milde Tensidgemische, enthaltend
a) 10 bis 40 Gew.-% eines Alkyl- und/oder Alkenyloligoglucosids der Formel **(I)**,
R¹-O-[G]ₚ (I)
in der
R¹ für einen Alkyl- und/oder Alkenylrest mit 8 bis 11 Kohlenstoffatomen,
[G] für einen Glucoserest und
p für Zahlen von 1 bis 10 steht,
b) 10 bis 40 Gew.-% eines Alkyl- und/oder Alkenyloligoglucosides der Formel **(II)**,
R²-O-[G]ₚ (II)
in der
R² für einen Alkyl- und/oder Alkenylrest mit 12 bis 22 Kohlenstoffatomen,
[G] für einen Glucoserest und
p für Zahlen von 1 bis 10 steht und
c) 80 bis 20 Gew.-% eines Alkylethersulfats der Formel **(III)**,
R³-(OCH₂CH₂)ₙO-SO₃M (III)
in der
R³ für einen Alkyl- und/oder Alkenylrest mit 6 bis 22 Kohlenstoffatomen,
M für ein Alkali-, Erdalkali-, Ammonium- und/oder Alkanolammoniumion und
n für Zahlen von 1 bis 20
steht.

2. Milde Tensidgenische nach Anspruch 1, **dadurch gekennzeichnet**, daß die Komponente a) Alkyloligoglucoside der Formel **(I)** darstellt, in der R¹ für Alkylreste mit 8 bis 10 Kohlenstoffatomen und p für Zahlen von 1 bis 3 steht.

3. Milde Tensidgenische nach Anspruch 1, **dadurch gekennzeichnet**, daß die Komponente a) Alkyloligoglucoside der Formel **(I)** darstellt, in der R¹ für Alkylreste mit 9 bis 11 Kohlenstoffatomen und p für Zahlen von 1 bis 3 steht.

4. Milde Tensidgenische nach Anspruch 1, **dadurch gekennzeichnet**, daß die Komponente b) Alkyloligoglucoside der Formel **(II)** darstellt, in der R² für Alkylreste mit 12 bis 14 Kohlenstoffatomen und p für Zahlen von 1 bis 3 steht.

5. Milde Tensidgenische nach Anspruch 1, **dadurch gekennzeichnet**, daß die Komponente c) Alkylethersulfate der Formel **(III)** darstellt, in der R³ für Alkylreste mit 12 bis 18 Kohlenstoffatomen, M für Natrium und/oder Magnesium und n für Zahlen von 2 bis 7 steht.

6. Milde Tensidgemische nach Anspruch 1, **dadurch gekennzeichnet**, daß die Komponenten a) und b) im Gewichtsverhältnis 1 : 1 bis 4 : 1 enthalten sind.

7. Milde Tensidgemische nach Anspruch 1, **dadurch gekennzeichnet**, daß die Komponenten (a+b) und c) im Gewichtsverhältnis 4 : 1 bis 1 : 4 enthalten sind.

8. Verwendung der milden Tensidgemische nach Anspruch 1 zur Herstellung von manuellen geschirrspülmitteln.

9. Verwendung der milden Tensidgemische nach Anspruch 1 zur Herstellung von kosmetischen Mitteln.

## Claims

1. Mild surfactant mixtures containing
a) about 10 to about 40% by weight of an alkyl and/or alkenyl oligoglucoside corresponding to formula **(I)**
R¹-O-[G]ₚ (I)
in which
R¹ is an alkyl and/or alkenyl radical containing about 8 to about 11 carbon atoms,
[G] is a glucose unit and
p is a number of 1 to about 10,
b) about 10 to about 40% by weight of an alkyl and/or alkenyl oligoglucoside corresponding to formula **(II)**
R²-O-[G]ₚ (II)
in which
R² is an alkyl and/or alkenyl radical containing about 12 to about 22 carbon atoms,
[G] is a glucose unit and
p is a number of 1 to about 10, and
c) about 80 to about 20% by weight of an alkyl ether sulfate corresponding to formula **(III)**
R³-(OCH₂CH₂)ₙO-SO₃M (III)
in which
R³ is an alkyl and/or alkenyl radical containing about 6 to about 22 carbon atoms,
M is an alkali metal, alkaline earth metal, ammonium and/or alkanolammonium ion and
n is a number of 1 to about 20.

2. Mild surfactant mixtures as claimed in claim 1, **characterised in that** component a) is formed by alkyl oligoglucosides corresponding to formula **(I)** in which R¹ represents alkyl radicals containing 8 to 10 carbon atoms and p is a number of 1 to about 3.

3. Mild surfactant mixtures as claimed in claim 1, **characterized in that** component a) is formed by alkyl oligoglucosides corresponding to formula **(I)**, in which R¹ represents alkyl radicals containing 9 to 11 carbon atoms and p is a number of 1 to about 3.

4. Mild surfactant mixtures as claimed in claim 1, **characterized in that** component b) is formed by alkyl oliglucosides corresponding to formula **(II)**, in which R² represents alkyl radicals containing about 12 to about 14 carbon atoms and p is a number of 1 to about 3.

5. Mild surfactant mixtures as claimed in claim 1, **characterized in that** component c) is formed by alkyl ether sulfates corresponding to formula **(III)**, in which R³ represents alkyl radicals containing about 12 to about 18 carbon atoms, M is sodium and/or magnesium and n is a number of 2 to about 7.

6. Mild surfactant mixtures as claimed in claim 1, **characterized in that** components a) and b) are present in a ratio by weight of about 1:1 to about 4:1.

7. Mild surfactant mixtures as claimed in claim 1, **characterised in that** components (a+b) and c) are present in a ratio by weight of about 4:1 to about 1:4.

8. The use of the mild surfactant mixtures claimed in claim 1 for the production of manual dishwashing detergents.

9. The use of the mild surfactant mixtures claimed in claim 1 for the production of cosmetic preparations.

## Revendications

1. Mélanges de tensioactifs doux contenant :
a) de 10 à 40 % en poids d'un alkyloligoglucoside et/ou alcényloligoglucoside de formule (I),
R¹-O-[G]p (I)
dans laquelle,
R¹ représente un radical alkyle et/ou alcényle avec 8 à 11 atomes de carbone (G) représente un radical glucose et,
p représente des nombres de 1 à 10
b) de 10 à 40 % en poids d'un alkyloligoglucoside et/ou alcényloligoglucoside de la formule (II),
R²-O-[G]p (II)
dans laquelle,
R² représente un radical alkyl et/ou un radical alcényl avec de 12 à 22 atomes de carbone,
(G) représente un radical glucose et,
p représente des nombres de 1 à 10 et,
c) de 80 à 20 % en poids d'un sulfate d'éther d'alkyle de la formule (III),
R³-(OCH₂CH₂)ₙO-SO₃M (III)
dans laquelle,
R³ représente un radical alkyl-et/ou un radical alcényl avec de 6 à 22 atomes de carbone,
M représente un ion alcalin, alcalino-terreux, d'ammonium et/ou d'alkanolammonium et,
n représente des nombres de 1 à 20.

2. Mélanges de tensioactifs doux selon la revendication 1, caractérisés en ce que,
le composant a) représente des alkyloligoglucosides de la formule (I), dans laquelle R¹ représente des radicaux alkyle ayant de 8 à 10 atomes de C et p des nombres de 1 à 3.

3. Mélanges de tensioactifs doux selon la revendication 1, caractérisés en ce que,
le composant a) représente des alkyloligoglucosides de la formule (I) dans laquelle R¹ représente des radicaux alkyle ayant de 9 à 11 atomes de carbone et p des nombres de 1 à 3.

4. Mélanges de tensioactifs doux selon la revendication 1, caractérisés en ce que,
le composant b) représente des alkyloligoglucosides de la formule (II), dans laquelle R² représente des radicaux alkyle ayant de 12 à 14 atomes de C et p des nombres de 1 à 3.

5. Mélanges de tensioactifs doux selon la revendication 1, caractérisés en ce que,
le composant c) représente des sulfates d'éther d'alkyle de la formule (III), dans laquelle R³ représente des radicaux alkyle ayant de 12 à 18 atomes de C, M du sodium et/ou du magnésium et n des nombres de 2 à 7.

6. Mélanges de tensioactifs doux selon la revendication 1, caractérisés en ce que,
les composants a) et b) sont contenus dans le rapport pondéral 1:1 à 4:1.

7. Mélanges de tensioactifs doux selon la revendication 1, caractérisés en ce que,
les composants (a+b) et c) sont contenus dans le rapport pondéral 4:1 à 1:4.

8. Utilisation des tensioactifs doux selon la revendication 1 pour la préparation de produits de lavage manuel de la vaisselle.

9. Utilisation des mélanges de tensioactifs doux selon la revendication 1 pour la préparation d'agents cosmétiques.
